# EUROPÄISCHE PATENTSCHRIFT

(11) **EP 3 561 487 B1**
(45) Veröffentlichungstag und Bekanntmachung des Hinweises auf die Patenterteilung: **18.01.2023**
(21) Anmeldenummer: 18169319.3
(22) Anmeldetag: 25.04.2018
(51) Int. Cl.: G01N 21/3504, G01N 21/17

(54) **MESSVORRICHTUNG ZUR ANALYSE EINER ZUSAMMENSETZUNG EINES BRENNGASES MIT EINER VOR EINEM DETEKTOR ANGEORDNETEN FILTERKAMMER**
MEASURING DEVICE FOR ANALYSIS OF A COMPOSITION OF A COMBUSTIBLE GAS WITH A FILTER CHAMBER ARRANGED IN FRONT OF A DETECTOR
DISPOSITIF DE MESURE DESTINÉ À L'ANALYSE D'UNE COMPOSITION D'UN GAZ DE COMBUSTION POURVU D'UNE CHAMBRE FILTRANTE DISPOSÉE DEVANT UN DÉTECTEUR

(43) Veröffentlichungstag der Anmeldung: 30.10.2019
(73) Patentinhaber: ABB Schweiz AG, 5400 Baden (CH)
(72) Erfinder: Wiedemann, Susanne, 63636 Brachttal (DE); Kleemann, Wolfgang, 61440 Oberursel (DE); Rathke, Carsten, 63450 Hanau (DE); Clavin, Matias-Hugo, 1824 Lanus Oeste (AR); Kappler, Jürgen, 60435 Frankfurt am Main (DE)
(74) Vertreter: Maiwald GmbH

(56) Entgegenhaltungen:
- EP-A1- 0 072 222
- EP-A1- 0 685 728
- WO-A1-2010/145809
- CH-A5- 685 889
- DE-A1- 3 522 949
- GB-A- 2 113 833
- JP-A- S60 149 949
- US-A- 4 027 972
- US-A- 4 058 725
- US-A- 5 077 469
- US-A- 5 292 666
- US-A- 6 006 585

## Beschreibung

Die Erfindung betrifft eine Messvorrichtung zur Analyse einer Zusammensetzung eines Brenngases, das zumindest ein erstes Gas und ein zweites von dem ersten verschiedenes Gas aufweist, wobei das erste Gas und das zweite Gas ein Infrarotlicht in zumindest einem gemeinsamen ersten Wellenlängenbereich im elektromagnetischen Spektrum absorbieren können.

Aus der US 4 027 972 A ist ein Verfahren und eine Vorrichtung zum Analysieren von Gas gekannt. Es wird dabei eine relative Gasmenge eines ersten und eines zweiten Gases eines unbekannten Gasgemischs mittels bekannter Gasprobenvolumen bestimmt. Zur Bestimmung der Gasmenge wird eine Strahlenergie einer bestimmten Wellenlänge in die hintereinander angeordneten Gasvolumenkörper eingebracht.

In der EP 0 685 7287 A1 ist eine Messvorrichtung zur Analyse einer Zusammensetzung eines Gases offenbart. Die Messvorrichtung arbeitet dabei nach dem photoakustischen Effekt, bei der das Licht einer pulsierenden Lichtquelle in mehrere Messkammern und mehrere Referenzkammern eingeleitet wird.

Derart bekannte Messvorrichtungen weisen häufig einen intermittierenden ersten Infrarotstrahler, eine erste Probenkammer zur Aufnahme des Brenngases, einen ersten Detektor, der zumindest das erste Gas enthält und nach dem photoakustischen Effekt arbeitet, auf. Dadurch, dass das erste Gas und das zweite Gas Infrarotlicht in dem ersten Wellenlängenbereich absorbieren, weist der erste Detektor gegenüber dem zweiten Gas eine Querempfindlichkeit auf. Diese Querempfindlichkeit kann zu einer ungenauen Messung einer Konzentration des ersten Gas in dem Brenngas mithilfe des ersten Detektors führen.

Ausgehend hiervon ist es die Aufgabe der vorliegenden Erfindung, eine Messvorrichtung der gattungsmäßigen Art dahingehend weiter zu verbessern, dass eine Querempfindlichkeit des ersten Detektors reduziert wird.

Diese Aufgabe wird mit einer Messvorrichtung mit den Merkmalen des Anspruches 1 und einem Verfahren mit den Merkmalen des Anspruches 9 gelöst. Vorteilhafte Ausgestaltungen der Messvorrichtung und Weiterbildungen des Verfahrens sind Gegenstand der abhängigen Ansprüche.

Mit der Analyse der Zusammensetzung ist vor allem eine Ermittlung von Massen- und/oder Stoffmengenverhältnissen zwischen dem ersten und dem zweiten Gas und bevorzugt weiteren Gasen, die in dem Brenngas enthalten sind, gemeint. Das Brenngas ist bevorzugt Erdgas, das verschiedene Kohlenwasserstoffverbindungen aufweist. So enthält das Brenngas zumindest das erste Gas, welches vorzugsweise Propan (C₃H₈) ist, und das zweite Gas, welches bevorzugt Methan (CH₄) ist. Der erste Wellenlängenbereich kann durch eine untere und eine obere Grenze definiert sein, wobei die beiden Grenzen durch einen Wert einer Wellenzahl, beispielsweise 2750 1/cm für die untere Grenze und 2900 1/cm für die obere Grenze, beschrieben werden können.

Der intermittierende erste Infrarotstrahler kann in einer ersten Variante eine Infrarotlichtquelle und einen im Strahlengang der Infrarotlichtquelle angeordneten mechanischen Pulsformer aufweisen. In einer zweiten Variante kann der erste Infrarotstrahler in Form eines gepulsten Lasers ausgebildet sein. In jedem Fall erzeugt der Infrarotstrahler eine Infrarotstrahlung, welche eine über ein Zeitintervall veränderliche Intensität aufweist. Die Intensität ändert sich vorzugsweise pulsartig von einem ersten niedrigen Niveau zu einem zweiten höheren Niveau. Der Infrarotstrahler strahlt die Infrarotstrahlung in einer Hauptrichtung ab, die von dem Infrarotstrahler in Richtung des ersten Detektors zeigt.

Die veränderliche Intensität bewirkt, dass das erste in dem ersten Detektor befindliche Gas eine sich über dem Zeitintervall ändernde Energiemenge von der Infrarotstrahlung absorbiert.

Dadurch wird in dem ersten Detektor eine sich über dem Zeitintervall ändernde Wärmemenge erzeugt, die einen über der Zeit veränderlichen Druck bewirkt. Dieser über der Zeit veränderliche Druck erzeugt Druckwellen, die der erste Detektor aufnimmt und die mit Hilfe eines Verstärkers der Messvorrichtung verstärkt und zu Signalen verarbeitet werden. Die Signale können mit Hilfe einer Auswertungseinheit derart ausgewertet werden, dass eine Relation zwischen einer Differenz einer Amplitude der über der Zeit schwankenden Druckwelle beziehungsweise des Signals und einer ersten Konzentration des ersten Gases in der ersten Probenkammer ermittelt werden kann. Der Effekt, dass durch Absorption von elektromagnetischer Strahlung eine Druckwelle erzeugt werden kann, wird als photoakustischer Effekt bezeichnet.

Dadurch, dass die Messvorrichtung die erste Filterkammer mit dem zweiten Gas aufweist, absorbiert das zweite Gas diejenigen elektromagnetischen Wellen der Infrarotstrahlung, die eine Energie haben, die ein Elektron eines Atoms eines Moleküls des zweiten Gases aufnimmt, wenn es von einem Grundzustand in einen angeregten Zustand überführt wird. Die vom Elektron aufgenommene Energie hängt unter anderem von einer Rotation und einer Schwingung der Moleküle des zweiten Gases ab. Im Gegensatz zu scharf definierten Energiebeträgen bei Atomen, liegen bei Molekülen viele absorbierbare Energiewerte dicht beieinander und bilden im Absorptionsspektrum breitere dunkle Bereiche, sogenannte Absorptionsbanden aus. Das erste und das zweite Gas weisen nun sich überlappende Absorptionsbanden auf.

Nachdem die Elektronen die Energie aufgenommen haben, geben die Elektronen die aufgenommene Energie wieder ab. Dies kann beispielsweise in Form von Wärme oder elektromagnetischer Strahlung erfolgen. Entscheidend ist, dass die von den Elektronen abgegebene Energie sich in alle Richtungen des Raumes verteilt, wodurch eine Abschwächung einer Intensität derjenigen elektromagnetischen Wellen, die die jeweiligen aufgenommenen Energien der Elektronen des zweiten Gases aufweisen, in Richtung der Hauptrichtung erzielt wird. Dies hat zur Folge, dass diese elektromagnetischen Wellen in dem ersten Detektor kaum noch detektiert werden können.

Die Konzentration des zweiten Gases in der ersten Filterkammer ist vorzugsweise bei verschiedenen Brenngasen, mit welchen die erste Probekammer gefüllt ist, konstant. Dadurch erfolgt die oben beschriebene Abschwächung der Intensität in der Hauptrichtung weitgehend unabhängig von einer Konzentration des zweiten Gases in dem Brenngas. Bei der vorgeschlagenen Messvorrichtung erfolgt die oben beschriebene Abschwächung im Wesentlichen durch das in der ersten Filterkammer befindliche zweite Gas und nicht durch das in der ersten Probenkammer befindliche zweite Gas. Des Weiteren erfolgt die Abschwächung auch für eine Intensität derjenigen Wellen, die innerhalb des ersten Wellenlängenbereiches liegen, d.h. die den sich überlappenden Absorptionsbanden entsprechen. Aus diesem Grund kann mithilfe der vorgeschlagenen Messvorrichtung eine Querempfindlichkeit des ersten Detektors gegenüber dem zweiten Gas reduziert werden.

Dadurch kann nicht nur erreicht werden, dass eine Druckwelle, die der erste Detektor zur Berechnung einer Konzentration des ersten Gases in dem Brenngas erfasst, in Abhängigkeit einer Konzentration des zweiten Gases in dem Brenngas verfälscht wird. Weiterhin kann eine Empfindlichkeit des ersten Detektors gegenüber derjenigen elektromagnetischen Strahlung erhöht werden, die vorzugsweise einem korrigierten Absorptionsspektrum des ersten Gases entspricht. Das korrigierte Absorptionsspektrum enthält bevorzugt nicht die sich überlappenden Absorptionsbanden. Dies ist dadurch begründet, dass eine gesamte elektromagnetische Energie, die auf den ersten Detektor trifft, durch die vor dem ersten Detektor angeordnete erste Filterkammer reduziert wird. So kann ein an dem ersten Detektor angeschlossener Verstärker im Vergleich zu einer Messvorrichtung, die nicht die erste Filterkammer aufweist, mit einem geringeren Vorwiderstand betrieben werden. Dabei kann der Vorwiderstand durch ein Justieren eines Potentiometers verstellbar sein. Durch die erhöhte Empfindlichkeit kann die Amplitude der Differenz des Signals erhöht werden, wodurch Konzentrationsunterschiede des zweiten Gases im Brenngas genauer erfasst werden können.

Eine vorteilhafte Ausgestaltung sieht vor, dass die Messvorrichtung eine erste Kalibrierungskammer zum Kalibrieren der Messvorrichtung aufweist. Die erste Kalibrierungskammer enthält ein Mischgas mit einem vorgegebenen Mengenverhältnis zwischen einer ersten Menge des ersten Gases und einer zweiten Menge des zweiten Gases und ist zwischen der ersten Probenkammer und der ersten Filterkammer positionierbar.

Das vorgegebene Mengenverhältnis liegt vorzugsweise in einem Bereich, in dem ein Mengenverhältnis zwischen der Menge des ersten Gases und der Menge des zweiten Gases in dem Brenngas liegt. Der Bereich kann beispielsweise durch einen unteren und einen oberen Grenzwert definiert sein, wobei diese Grenzwerte durch Sicherheitsvorschriften vorgegeben sein können. Die Kalibrierungskammer ist beweglich in der Messvorrichtung angeordnet. Dies hat den Zweck, dass die Kalibrierungskammer zwischen dem Infrarotstrahler und dem ersten Detektor in eine erste Position bewegt werden kann, wenn sich in der ersten Probenkammer kein Brenngas befindet. In einem solchen Zustand der Messvorrichtung kann der Verstärker derart justiert werden, dass mit Hilfe des ersten Detektors eine Absorption von elektromagnetischen Wellen, die innerhalb des korrigierten Absorptionsspektrums liegen, mit einer größtmöglichen Empfindlichkeit einer Gesamteinheit, die durch den ersten Detektor und den Verstärker gebildet wird, erfasst werden kann.

Vorzugsweise weist die Messvorrichtung eine weitere Kammer auf, die mit der Kalibrierungskammer mechanisch fest verbunden ist. Dies hat den Vorteil, dass sich die weitere Kammer zwischen den ersten Infrarotstrahler und den ersten Detektor bewegen lässt, wenn die erste Kalibrierungskammer die erste Position verlässt. Die weitere Kammer enthält vorzugsweise Stickstoff.

In einer bevorzugten Ausführungsform ist die erste Filterkammer als ein zweiter Detektor ausgebildet, der nach dem photoakustischen Effekt arbeitet. Dies hat den Vorteil, dass die erste Filterkammer nicht nur eine Reduktion der Querempfindlichkeit des ersten Detektors bewirkt, sondern mit der ersten Filterkammer zusätzlich eine Konzentration des zweiten Gases in dem Brenngas ermittelt werden kann. Der zweite Detektor funktioniert bevorzugt nach dem gleichen Prinzip wie der erste Detektor, mit dem Unterschied, dass der zweite Detektor das zweite Gas anstatt das erste Gas enthält.

Die erste Probenkammer hat einen Fluideingang und einen Fluidausgang, wobei die Messvorrichtung während eines Durchströmens der ersten Probenkammer mit dem Brenngas betreibbar ist. Dadurch, dass die Messvorrichtung während des Durchströmens der ersten Probenkammer mit dem Brenngas betreibbar ist, kann die erste Probenkammer in einer Zugangsleitung für das Brenngas zu einem Brenner oder in einer Abzweigung einer solchen Zugangsleitung angeordnet werden. Hierdurch ist eine permanente Messung der Zusammensetzung des Brenngases möglich, wodurch der Brenner in Abhängigkeit von der erfassten Zusammensetzung des Brenngases mit Hilfe der vorgeschlagenen Messvorrichtung geregelt werden kann. Dies ermöglicht eine optimierte Verbrennung in dem Brenner und macht auch eine genaue Berechnung von Kosten des Brenngases möglich.

In einer Weiterbildung der Erfindung ist vorgesehen, dass das Brenngas zumindest ein drittes Gas und ein viertes von dem dritten verschiedenes Gas aufweist, wobei das dritte Gas und das vierte Gas ein Infrarotlicht in zumindest einem gemeinsamen zweiten Wellenlängenbereich im elektromagnetischen Spektrum absorbieren können. Die Messvorrichtung weist gemäß dieser Weiterbildung einen intermittierenden zweiten Infrarotstrahler, eine zweite Probenkammer zur Aufnahme des Brenngases, einen dritten Detektor, der zumindest das dritte Gas enthält und nach dem photoakustischen Effekt arbeitet, und eine zweite Filterkammer, die das vierte Gas enthält, auf. Die zweite Probenkammer, der dritte Detektor und die zweite Filterkammer sind derart zueinander angeordnet, dass ein von dem zweiten Infrarotstrahler ausgesandtes Infrarotlicht durch die zweite Probenkammer und die zweite Filterkammer verlaufen und auf den dritten Detektor treffen kann und das Brenngas von dem Fluidausgang der ersten Probenkammer zu einem Fluideingang der zweiten Probenkammer geleitet werden kann.

Diese Weiterbildung der Messvorrichtung hat den Vorteil, dass Anteile, insbesondere Massen- oder Stoffmengenanteile, des ersten und dritten Gases in dem Brenngas ermittelt werden können. Das dritte Gas ist vorzugsweise Ethan und das vierte Gas bevorzugt Propan.

In einer weiteren Ausgestaltung ist vorgesehen, dass die Messvorrichtung einen vierten Detektor aufweist, der zwischen dem zweiten Infrarotstrahler und dem dritten Detektor angeordnet ist und ein fünftes Gas aufweist. Mit dieser Ausgestaltung kann eine kombinierte Messung einer Konzentration des dritten und fünften Gases durchgeführt werden, wobei zusätzlich die Querempfindlichkeit des dritten Detektors gegenüber dem vierten Gas reduziert wird. Das fünfte Gas ist bevorzugt Kohlendioxid.

Zur Lösung der Aufgabe wird weiterhin ein Verfahren zur Analyse eines Brenngases mithilfe einer Messvorrichtung nach einer der oben beschriebenen Ausgestaltungen vorgeschlagen. Das Verfahren hat die folgenden Schritte. In einem ersten Schritt wird das Brenngas in die erste Probenkammer eingeleitet. In einem zweiten Schritt wird mit dem ersten Infrarotstrahler eine intermittierende Infrarotstrahlung erzeugt. In einem dritten Schritt wird eine erste Intensität der Infrarotstrahlung mit dem ersten Detektor detektiert. In einem vierten Schritt wird zumindest eine Konzentration des ersten Gases in Abhängigkeit der ersten Intensität der Infrarotstrahlung ermittelt.

Das Verfahren kann vorteilhaft die folgenden weiteren Schritte umfassen. In einem sechsten Schritt kann eine zweite Intensität der Infrarotstrahlung mit dem zweiten Detektor erfasst werden. In einem siebten Schritt kann eine zweite Konzentration des zweiten Gases in Abhängigkeit der zweiten Intensität der Infrarotstrahlung ermittelt werden. Die Nummerierung der einzelnen Schritte gibt keine notwendige Reihenfolge des Verfahrens vor. So ist es möglich, die zweite Konzentration vor der ersten Konzentration ermittelt werden.

Im Rahmen der oben beschriebenen Weiterbildung der Messvorrichtungen ist es genauso gut möglich, dass das Verfahren durch folgende weitere Schritte ergänzt wird. So kann in einem achten Schritt das Brenngas von der ersten Probenkammer in die zweite Probenkammer geleitet und in einem neunten Schritt eine intermittierende Infrarotstrahlung mit dem zweiten Infrarotstrahler erzeugt werden. Ein zehnter Schritt kann vorsehen, eine dritte Intensität der Infrarotstrahlung mit dem dritten Detektor zu erfassen. In einem elften Schritt wird bevorzugt eine dritte Konzentration des dritten Gases in Abhängigkeit der dritten Intensität der Infrarotstrahlung ermittelt. Bevorzugt wird in einem zwölften Schritt eine vierte Intensität der Infrarotstrahlung mit der zweiten Filterkammer detektiert, wobei die zweite Filterkammer als ein vierter Detektor ausgebildet ist. Ein dreizehnter Schritt kann vorsehen, eine vierte Konzentration des vierten Gases in Abhängigkeit der vierten Intensität der Infrarotstrahlung zu ermitteln.

In Abhängigkeit der ersten, zweiten, dritten und/oder vierten Konzentration wird bevorzugt ein Brennwert, ein Heizwert, ein unterer Wobbeindex und/oder ein oberer Wobbeindex des Brenngases bestimmt.

Weitere Vorteile, Merkmale und Einzelheiten der Erfindung ergeben sich aus der nachfolgenden Beschreibung sowie anhand der Figuren. Dabei bezeichnet ein mehrfach verwendetes Bezugszeichen dieselbe Komponente. Die Figuren zeigen schematisch in:
- Figur 1: eine Messvorrichtung zur Analyse einer Zusammensetzung eines Brenngases mithilfe einer ersten Filterkammer,
- Figur 2: eine weitere Messvorrichtung zur Analyse einer Zusammensetzung eines Brenngases mithilfe einer ersten Filterkammer,
- Figur 3: eine weitere Messvorrichtung zur Analyse einer Zusammensetzung eines Brenngases mithilfe von zwei Filterkammern,
- Figur 4: eine weitere Messvorrichtung zur Analyse einer Zusammensetzung eines Brenngases mithilfe von zwei Filterkammern und vier Detektoren.

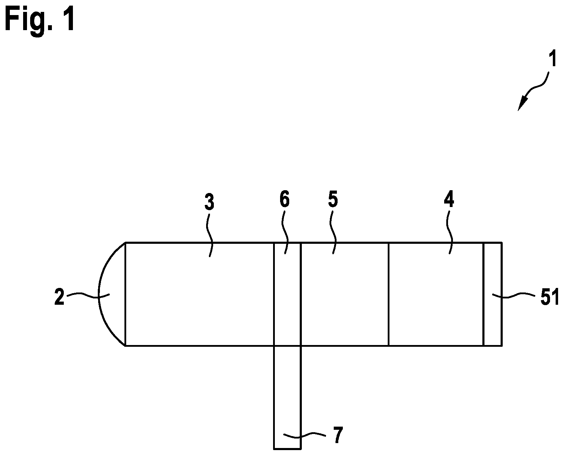

Fig. 1 zeigt eine Messvorrichtung **1** zur Analyse einer Zusammensetzung eines Brenngases, das zumindest ein erstes Gas, vorzugsweise Propan, und ein zweites von dem ersten verschiedenes Gas, vorzugsweise Methan, aufweist. Das erste und das zweite Gas können ein Infrarotlicht in zumindest einem gemeinsamen ersten Wellenlängenbereich im elektromagnetischen Spektrum absorbieren. Die Messvorrichtung **1** hat weiterhin einen intermittierenden ersten Infrarotstrahler **2,** eine erste Probenkammer **3** zur Aufnahme des Brenngases, einen ersten Detektor **4** und eine erste Filterkammer **5.** Der erste Detektor **4** weist zumindest das erste Gas auf und arbeitet nach dem photoakustischen Effekt. Die erste Filterkammer 5 enthält das zweite Gas und ist zwischen dem ersten Detektor **4** und dem ersten Infrarotstrahler **2** angeordnet. Dadurch kann ein von dem ersten Infrarotstrahler **2** ausgesandtes Infrarotlicht zuerst durch die erste Probekammer **3,** anschließend durch die erste Filterkammer 5 verlaufen und danach auf den ersten Detektor **4** treffen.

Die in Fig. 1 gezeigte Messvorrichtung **1** zeigt eine spezielle Ausgestaltung der vorgeschlagenen Messvorrichtung, bei der die Messvorrichtung **1** zusätzlich eine Kalibrierungskammer **6** aufweist. Die Kalibrierungskammer **6** ist verschiebbar zwischen der ersten Probenkammer **3** und der ersten Filterkammer **5** angeordnet. Die Kalibrierungskammer 6 kann derart verschoben werden, dass sie in einer in Fig. 1 gezeigten ersten Position zwischen der ersten Probenkammer **3** und der ersten Filterkammer **5** angeordnet ist. Darüber hinaus kann die Kalibrierungskammer 6 derart bewegt werden, dass sie sich in einer in Fig. 1 nicht dargestellten zweiten Position befindet, in der ein in der Kalibrierungskammer **6** enthaltenes Mischgas nicht von einem Infrarotstrahl getroffen wird, der von dem ersten Infrarotstrahler **2** in Richtung des ersten Detektors **4** ausgesandt wird. Das Mischgas weist ein vorgegebenes Mengenverhältnis zwischen einer ersten Menge des ersten Gases und einer zweiten Menge des zweiten Gases auf.

Vorzugsweise ist eine Leerkammer **7** zwischen der ersten Probenkammer **3** und der ersten Filterkammer **5** angeordnet, wenn die Kalibrierungskammer **6** sich in der zweiten Position befindet. Die Leerkammer **7** enthält vorzugsweise Stickstoff. Eine spezielle Ausgestaltung der Messvorrichtung **1** sieht vor, dass die erste Filterkammer **5** in Form eines zweiten Detektors ausgebildet ist, der nach dem photoakustischen Effekt arbeitet und mit dem eine Konzentration des zweiten Gases in dem Brenngas ermittelt werden kann. Der zweite Detektor ist vorzugsweise ähnlich wie der oben beschriebene erste Detektor ausgebildet, wobei der zweite Detektor das zweite Gas anstatt das erste Gas aufweist.

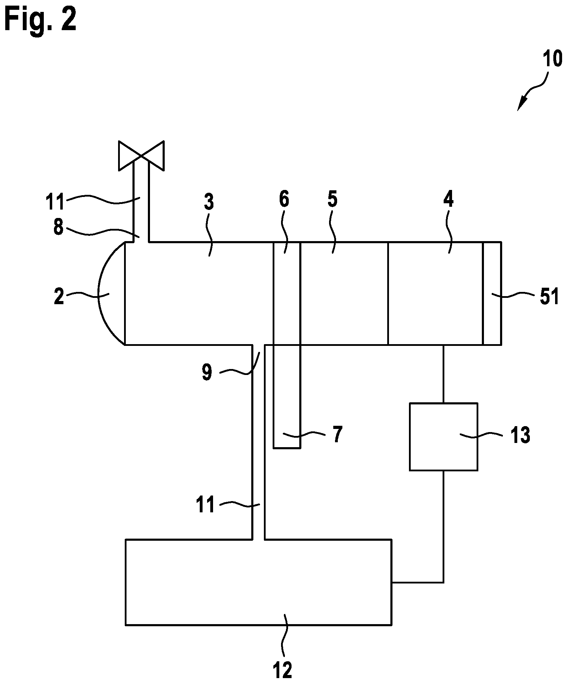

Fig. 2 zeigt eine weitere Messvorrichtung **10,** die die gleichen Komponenten wie die in Fig. 1 gezeigte Messvorrichtung **1** aufweist. Zusätzlich hat die erste Probenkammer **3** einen Fluideingang **8** und einen Fluidausgang **9,** wodurch die Messvorrichtung während eines Durchströmens der ersten Probenkammer **3** mit dem Brenngas betreibbar ist.

In Fig. 2 ist weiterhin eine Möglichkeit dargestellt, bei welcher die erste Probenkammer **3** in einen Zulauf **11** für das Brenngas eines Brenners **12** geschaltet ist, sodass das Brenngas für den Brenner **12** die Probenkammer **3** durchströmt. Dadurch kann das durch den Zulauf **11** zu dem Brenner **12** geleitete Brenngas permanent mit Hilfe der Messvorrichtung **10** analysiert werden. Die Analyse umfasst in vorteilhafter Weise eine Ermittlung eines unteren und/oder oberen Brennwertes und eines unteren und/oder oberen Wobbe-Index, wobei diese Werte vorzugsweise mit Hilfe einer Auswertungseinheit **13** berechnet werden. Diese errechneten Werte können dazu benutzt werden, um den Brenner **12** zu regeln. Hierzu sendet die Auswertungseinheit **13** die berechneten Werte an ein Steuergerät des Brenners **12.** Des Weiteren können diese Werte auch für eine Berechnung eines Preises des Brenngases verwendet werden.

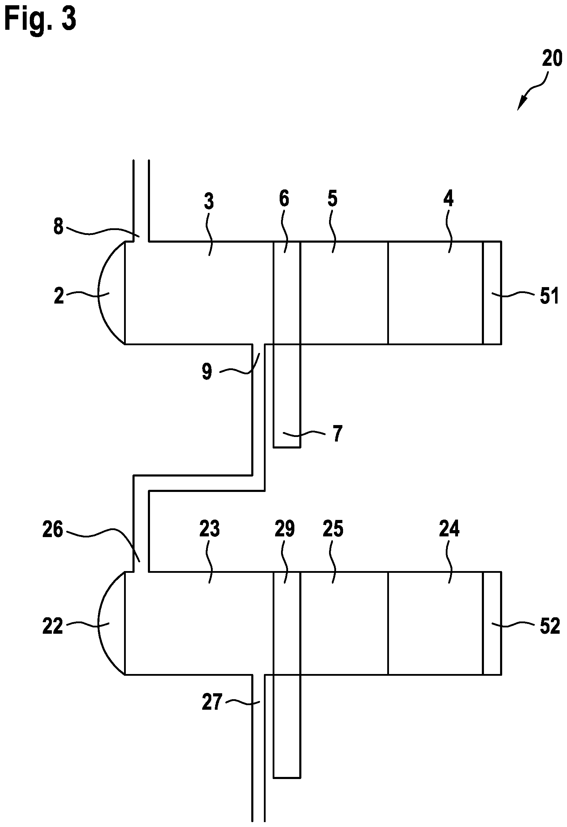

Fig. 3 zeigt eine weitere Messvorrichtung **20,** die die gleichen Komponenten wie die in Fig. 2 gezeigte Messvorrichtung **10** aufweist. Zusätzlich weist die Messvorrichtung **20** einen intermittierenden zweiten Infrarotstrahler **22,** eine zweite Probenkammer **23** zur Aufnahme des Brenngases, einen dritten Detektor **24,** der zumindest ein drittes Gas, vorzugsweise Ethan, aufweist und nach dem photoakustischen Effekt arbeitet, und eine zweite Filterkammer **25,** die in viertes Gas, bevorzugt Propan, enthält, auf. Die zweite Probenkammer **23,** der dritte Detektor **24** und die zweite Filterkammer **25** sind derart zueinander angeordnet, dass ein von dem zweiten Infrarotstrahler **22** ausgesandtes Infrarotlicht durch die zweite Probenkammer **23** und die zweite Filterkammer **25** verlaufen und auf den dritten Detektor **24** treffen kann. Des Weiteren kann das Brenngas von dem Fluidausgang **9** der ersten Probenkammer **3** zu einem Fluideingang **26** der zweiten Probenkammer **23** geleitet werden. Das Brenngas kann die zweite Probenkammer **23** über einen Fluidausgang **27** der zweiten Probenkammer verlassen. Fig. 3 stellt eine Variante dar, bei der die Messvorrichtung **20** eine zweite Kalibrierungskammer **29** hat, die zwischen der zweiten Probenkammer **23** und der zweiten Filterkammer **25** angeordnet ist. Die zweite Kalibrierungskammer **29** enthält bevorzugt ein Mischgas, welches das dritte und das vierte Gas aufweist, wobei ein Mischungsverhältnis zwischen dem dritten und dem vierten Gas bekannt ist.

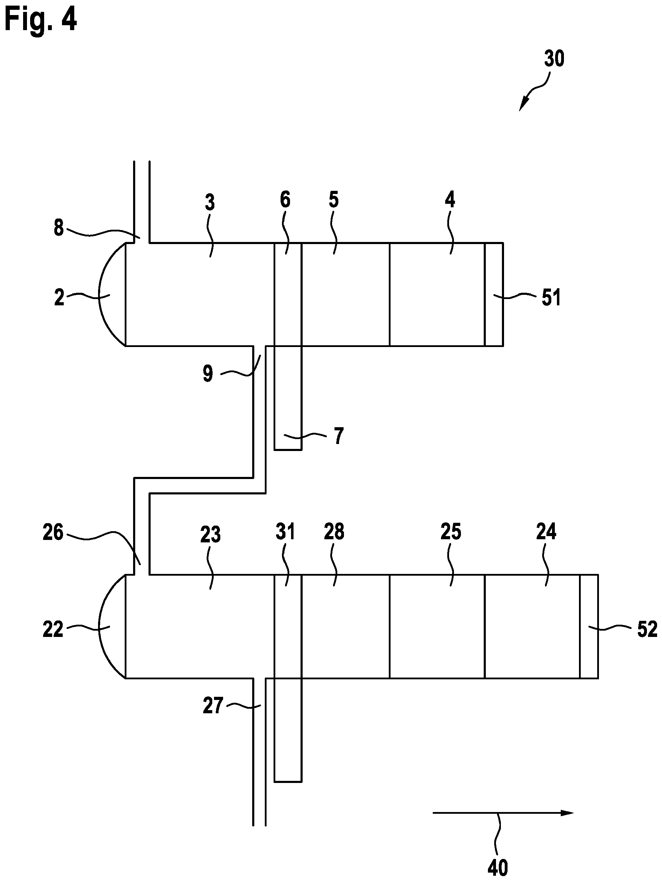

Fig. 4 zeigt eine weitere Messvorrichtung **30,** die die gleichen Komponenten wie die in Fig. 3 gezeigte Messvorrichtung **20** aufweist. Zusätzlich hat die Messvorrichtung **20** einen vierten Detektor **28** aufweist, der zwischen dem zweiten Infrarotstrahler **22** und dem dritten Detektor **24** angeordnet ist und ein fünftes Gas, bevorzugt Kohlendioxid, aufweist. Mithilfe des vierten Detektors kann eine Konzentration des fünften Gases in dem Brenngas erfasst werden. Ferner zeigt Fig. 4 eine spezielle Ausgestaltung der Messvorrichtung **30,** bei der die Messvorrichtung **30** eine zweite Kalibrierungskammer **31** hat, die zwischen der zweiten Probenkammer **23** und dem vierten Detektor **28** angeordnet ist. Die zweite Kalibrierungskammer **31** enthält bevorzugt ein Mischgas, welches das dritte und das fünfte Gas aufweist, wobei ein Mischungsverhältnis zwischen dem dritten und dem fünften Gas bekannt ist.

Die Kalibrierungskammern **6, 29** und **31** funktionieren in gleiche Art wie die oben beschriebenen erste Kalibrierungskammer, mit dem Unterschied, dass die jeweiligen Kalibrierungskammern **6, 29** und **31** mit unterschiedlichen Gasen gefüllt sind.

Alle in Fig. 1 bis 4 dargestellten Grenzen sämtlicher Kammern sind in einer horizontalen Richtung **40** für die von dem ersten oder zweiten Strahler ausgesandte Infrarotstrahlung durchlässig. Die Messvorrichtungen blockieren die Infrarotstrahlung in der Richtung **40** an ihrem jeweiligen Ende mithilfe einer Abschlussscheibe **51, 52.**

### Bezugszeichenliste

- 1,10,20,30: Messvorrichtung
- 2,22: Infrarotstrahler
- 3,23: Probenkammer
- 4,24,28: Detektor
- 5,25: Filterkammer
- 6,29,31: Kalibrierungskammer
- 7: Leerkammer
- 8,26: Fluideingang
- 9,27: Fluidausgang
- 11: Zulauf
- 12: Brenner
- 13: Auswertungseinheit
- 40: horizontalen Richtung
- 51,52: Abschlussscheibe

## Patentansprüche

1. Messvorrichtung (1; 10; 20; 30) zur Analyse einer Zusammensetzung eines Brenngases, das zumindest ein erstes Gas und ein zweites von dem ersten verschiedenes Gas aufweist, wobei das erste Gas und das zweite Gas ein Infrarotlicht in zumindest einem gemeinsamen ersten Wellenlängenbereich im elektromagnetischen Spektrum absorbieren und die Messvorrichtung (1; 10; 20; 30) einen intermittierenden ersten Infrarotstrahler (2), eine erste Probenkammer (3) zur Aufnahme des Brenngases und einen ersten Detektor (4) umfasst, der zumindest das erste Gas aufweist und nach dem photoakustischen Effekt arbeitet, wobei die Messvorrichtung (1; 10; 20; 30) außerdem eine erste Filterkammer (5) aufweist, die das zweite Gas enthält, und die erste Probenkammer (3), der erste Detektor (4) und die erste Filterkammer (5) derart zueinander angeordnet sind, dass ein von dem ersten Infrarotstrahler (2) ausgesandtes Infrarotlicht durch die erste Probenkammer (3) und die erste Filterkammer (5) verlaufen und auf den ersten Detektor (4) treffen, wobei die Messvorrichtung (1; 10; 20; 30) eine erste Kalibrierungskammer (6) zum Kalibrieren der Messvorrichtung (1; 10; 20; 30) aufweist, wobei die erste Kalibrierungskammer ein Mischgas mit einem vorgegebenen Mengenverhältnis zwischen einer ersten Menge des ersten Gases und einer zweiten Menge des zweiten Gases enthält und zwischen der ersten Probenkammer (3) und der ersten Filterkammer (5) positionierbar ist, wobei die Kalibrierungskammer (6; 29; 31) beweglich in der Messvorrichtung (1; 10; 20; 30) angeordnet ist, wobei die erste Probenkammer (3) einen Fluideingang (8) und einen Fluidausgang (8) hat und die Messvorrichtung (10; 20; 30) während eines Durchströmens der ersten Probenkammer (3) mit dem Brenngas betreibbar ist, und die erste Probenkammer (3) in einem Zulauf (11) für das Brenngas eines Brenners (12) geschaltet ist, so dass das Brenngas für den Brenner (12) die Probenkammer (3) durchströmt, wobei eine Auswertungseinheit (13) vorgesehen ist, über welche die berechneten Werte zur Regelung des Brenners (12) an ein Steuergerät des Brenners (12) übermittelbar sind.

2. Messvorrichtung (1; 10; 20; 30) nach Anspruch 1,
**dadurch gekennzeichnet, dass**
die erste Filterkammer (5) als ein zweiter Detektor ausgebildet ist, der nach dem photoakustischen Effekt arbeitet.

3. Messvorrichtung (20; 30) nach einem der vorhergehenden Ansprüche,
**dadurch gekennzeichnet, dass**
das Brenngas zumindest ein drittes Gas und ein viertes von dem dritten verschiedenes Gas aufweist, wobei das dritte Gas und das vierte Gas ein Infrarotlicht in zumindest einem gemeinsamen zweiten Wellenlängenbereich im elektromagnetischen Spektrum absorbieren können und die Messvorrichtung (20; 30) einen intermittierenden zweiten Infrarotstrahler (22), eine zweite Probenkammer (23) zur Aufnahme des Brenngases, einen dritten Detektor (24), der zumindest das dritte Gas aufweist und nach dem photoakustischen Effekt arbeitet, und eine zweite Filterkammer (25), die das vierte Gas enthält, aufweist und die zweite Probenkammer (23), der dritte Detektor (24) und die zweite Filterkammer (25) derart zueinander angeordnet sind, dass ein von dem zweiten Infrarotstrahler (22) ausgesandtes Infrarotlicht durch die zweite Probenkammer (23) und die zweite Filterkammer (25) verlaufen und auf den dritten Detektor (24) trifft und das Brenngas von dem Fluidausgang der ersten Probenkammer (3) zu einem Fluideingang der zweiten Probenkammer (23) leitbar ist.

4. Messvorrichtung (30) nach Anspruch 3,
**dadurch gekennzeichnet, dass**
die Messvorrichtung (30) einen vierten Detektor (28) aufweist, der zwischen dem zweiten Infrarotstrahler (22) und dem dritten Detektor (24) angeordnet ist und ein fünftes Gas aufweist.

5. Messvorrichtung (1; 10; 20; 30) nach einem der vorhergehenden Ansprüche,
**dadurch gekennzeichnet, dass**
das erste Gas Propan und das zweite Gas Methan ist.

6. Messvorrichtung (1; 10; 20; 30) nach einem der vorhergehenden Ansprüche,
**dadurch gekennzeichnet, dass**
das dritte Gas Ethan und das vierte Gas Propan ist.

7. Messvorrichtung (1; 10; 20; 30) nach einem der vorhergehenden Ansprüche,
**dadurch gekennzeichnet, dass**
das fünfte Gas Kohlendioxid ist.

8. Verfahren zur Analyse eines Brenngases mithilfe der Messvorrichtung (1; 10; 20; 30) nach einem der Ansprüche 1 bis 7 mit den folgenden Schritten:
- Einleiten des Brenngases in die erste Probenkammer (3),
- Erzeugen einer intermittierenden Infrarotstrahlung mit dem ersten Infrarotstrahler (2),
- Detektieren einer ersten Intensität der Infrarotstrahlung mit dem ersten Detektor (4),
- Ermitteln zumindest einer Konzentration des ersten Gases in Abhängigkeit der ersten Intensität der Infrarotstrahlung,
- Übermitteln der berechneten Werte an ein Steuergerät des Brenners (12) zur Regelung des Brenners (12).

9. Verfahren nach Anspruch 8 mit einer Messvorrichtung (1; 10; 20; 30) nach einem der Ansprüche 1 bis 7 mit den folgenden zusätzlichen Schritten:
- Detektieren einer zweiten Intensität der Infrarotstrahlung mit dem zweiten Detektor,
- Ermitteln zumindest einer zweiten Konzentration des zweiten Gases in Abhängigkeit der zweiten Intensität der Infrarotstrahlung.

10. Verfahren nach Anspruch 8 oder 9 mit den folgenden zusätzlichen Schritten:
- Einleiten des Brenngases von der ersten Probenkammer (3) in die zweite Probenkammer (23),
- Erzeugen einer intermittierenden Infrarotstrahlung mit dem zweiten Infrarotstrahler (22),
- Detektieren einer dritten Intensität der Infrarotstrahlung mit dem dritten Detektor (24),
- Ermitteln zumindest einer dritten Konzentration des dritten Gases in Abhängigkeit der dritten Intensität der Infrarotstrahlung,
- Detektieren einer vierten Intensität der Infrarotstrahlung mit der zweiten Filterkammer (25), wobei die zweite Filterkammer (25) als ein vierter Detektor ausgebildet ist,
- Ermitteln zumindest einer vierten Konzentration des vierten Gases in Abhängigkeit der vierten Intensität der Infrarotstrahlung.

11. Verfahren nach einem der Ansprüche 8, 9 oder 10, wobei in Abhängigkeit der ersten, zweiten, dritten und/oder vierten Konzentration ein Brennwert oder ein Heizwert des Brenngases bestimmt wird.

12. Verfahren nach einem der Ansprüche 8 bis 11, wobei in Abhängigkeit der ersten, zweiten, dritten und/oder vierten Konzentration ein unterer oder oberer Wobbeindex des Brenngases bestimmt wird.

## Claims

1. A measuring device (1; 10; 20; 30) for analysis a composition of a combustible gas comprising at least a first gas and a second gas different from the first gas, the first gas and the second gas absorbing an infrared light in at least a common first wavelength range in the electromagnetic spectrum, the measuring device (1; 10; 20; 30) comprising an intermittent first infrared emitter (2), a first sample chamber (3) for containing the combustible gas and a first detector (4) comprising at least the first gas and operating according to the photoacoustic effect, wherein
the measuring device (1; 10; 20; 30) further comprises a first filter chamber (5) containing the second gas, and the first sample chamber (3), the first detector (4) and the first filter chamber (5) are arranged relative to each other such that an infrared light emitted from the first infrared emitter (2) passes through the first sample chamber (3) and the first filter chamber (5) and impinges on the first detector (4), wherein the measuring device (1; 10; 20; 30) comprises a first calibration chamber (6) for calibrating the measuring device (1; 10; 20; 30), the first calibration chamber containing a mixed gas with a predetermined quantity ratio between a first quantity of the first gas and a second quantity of the second gas and being positionable between the first sample chamber (3) and the first filter chamber (5), wherein the calibration chamber (6; 29; 31) being movably arranged in the measuring device (1; 10; 20; 30), wherein the first sample chamber (3) has a fluid inlet (8) and a fluid outlet (8) and the measuring device (10; 20; 30) can be operated while the combustible gas is flowing through the first sample chamber (3), and the first sample chamber (3) is connected in an inlet (11) for the combustible gas of a burner (12), so that the combustible gas for the burner (12) flows through the sample chamber (3), an evaluation unit (13) being provided, via which the calculated values can be transmitted to a control unit of the burner (12) for regulating the burner (12).

2. Measuring device (1; 10; 20; 30) according to claim 1,
**characterised in that**
the first filter chamber (5) is designed as a second detector which operates according to the photoacoustic effect.

3. Measuring device (20; 30) according to one of the preceding claims,
**characterized in that**
the combustible gas comprises at least a third gas and a fourth gas different from the third gas, the third gas and the fourth gas being capable of absorbing an infrared light in at least a common second wavelength range in the electromagnetic spectrum, and the measuring device (20; 30) comprises an intermittent second infrared emitter (22), a second sample chamber (23) for containing said combustible gas, a third detector (24) comprising at least said third gas and operating according to the photoacoustic effect, and a second filter chamber (25) containing said fourth gas, and said second sample chamber (23), the third detector (24) and the second filter chamber (25) are arranged with respect to each other in such a way that an infrared light emitted by the second infrared emitter (22) passes through the second sample chamber (23) and the second filter chamber (25) and impinges on the third detector (24), and the combustible gas can be conducted from the fluid outlet of the first sample chamber (3) to a fluid inlet of the second sample chamber (23).

4. Measuring device (30) according to claim 3,
**characterised in that**
the measuring device (30) comprises a fourth detector (28) arranged between the second infrared emitter (22) and the third detector (24) and comprising a fifth gas.

5. Measuring device (1; 10; 20; 30) according to any one of the preceding claims, **characterized in that**
the first gas is propane and the second gas is methane.

6. Measuring device (1; 10; 20; 30) according to any one of the preceding claims, **characterized in that**
the third gas is ethane and the fourth gas is propane.

7. Measuring device (1; 10; 20; 30) according to any one of the preceding claims, **characterized in that**
the fifth gas is carbon dioxide.

8. A method of analysing a combustible gas by means of the measuring device (1; 10; 20; 30) according to any one of claims 1 to 7, comprising the following steps:
- Introducing the combustible gas into the first sample chamber (3),
- generating an intermittent infrared radiation with the first infrared emitter (2),
- detecting a first intensity of the infrared radiation with the first detector (4),
- determining at least one concentration of the first gas as a function of the first intensity of the infrared radiation,
- transmitting the calculated values to a control unit of the burner (12) for controlling the burner (12).

9. A method according to claim 8, comprising a measuring device (1; 10; 20; 30) according to any one of claims 1 to 7, comprising the following additional steps:
- Detecting a second intensity of infrared radiation with the second detector,
- determining at least a second concentration of the second gas as a function of the second intensity of the infrared radiation.

10. A method according to claim 8 or 9 comprising the following additional steps:
- Introducing the combustible gas from the first sample chamber (3) into the second sample chamber (23),
- Generating an intermittent infrared radiation with the second infrared emitter (22),
- detecting a third intensity of the infrared radiation with the third detector (24),
- determining at least a third concentration of the third gas as a function of the third intensity of the infrared radiation,
- detecting a fourth intensity of the infrared radiation with the second filter chamber (25), wherein the second filter chamber (25) is formed as a fourth detector,
- determining at least a fourth concentration of the fourth gas as a function of the fourth intensity of the infrared radiation.

11. Method according to any one of claims 8, 9 or 10, wherein a calorific value or a heating value of the combustible gas is determined in dependence on the first, second, third and/or fourth concentration.

12. Method according to any one of claims 8 to 11, wherein a lower or upper Wobbe index of the fuel gas is determined as a function of the first, second, third and/or fourth concentration.

## Revendications

1. Dispositif de mesure (1 ; 10 ; 20 ; 30) destiné à l'analyse d'une composition d'un gaz combustible qui comporte au moins un premier gaz et un deuxième gaz différent du premier gaz, le premier gaz et le deuxième gaz absorbant la lumière infrarouge dans au moins une première gamme de longueurs d'onde commune dans le spectre électromagnétique et le dispositif de mesure (1 ; 10 ; 20 ; 30) comprenant une première source de rayonnement infrarouge intermittent (2), une première chambre d'échantillon (3) destinée à recevoir le gaz combustible et un premier détecteur (4) qui comporte au moins le premier gaz et qui fonctionne selon l'effet photoacoustique,
le dispositif de mesure (1 ; 10 ; 20 ; 30) comportant en outre une première chambre de filtrage (5) qui contient le deuxième gaz et la première chambre d'échantillon (3), le premier détecteur (4) et la première chambre de filtrage (5) étant disposés l'un par rapport à l'autre de manière à ce qu'une lumière infrarouge émise par la première source de rayonnement infrarouge (2) traverse la première chambre d'échantillon (3) et la première chambre de filtrage (5) et est incidente au premier détecteur (4), le dispositif de mesure (1 ; 10 ; 20 ; 30) comportant une première chambre d'étalonnage (6) destinée à étalonner le dispositif de mesure (1 ; 10 ; 20 ; 30), la première chambre d'étalonnage contenant un gaz de mélange dont le rapport de quantité spécifié est compris entre une première quantité du premier gaz et une deuxième quantité du deuxième gaz et pouvant être positionnée entre la première chambre d'échantillon (3) et la première chambre de filtrage (5), la chambre d'étalonnage (6 ; 29 ; 31) étant disposée de manière mobile dans le dispositif de mesure (1 ; 10 ; 20 ; 30), la première chambre d'échantillon (3) comportant une entrée de fluide (8) et une sortie de fluide (8) et le dispositif de mesure (10 ; 20 ; 30) pouvant être utilisé pendant que le gaz combustible s'écoule à travers la première chambre d'échantillon (3) et la première chambre d'échantillon (3) étant montée dans une amenée (11) du gaz combustible d'un brûleur (12) de sorte que le gaz combustible destiné au brûleur (12) s'écoule à travers la chambre d'échantillon (3), une unité d'évaluation (13) étant prévue qui permet de transmettre les valeurs calculées, destinées à la régulation du brûleur (12), à une unité de commande du brûleur (12).

2. Dispositif de mesure (1 ; 10 ; 20 ; 30) selon la revendication 1,
**caractérisé en ce que**
la première chambre de filtrage (5) est conçue comme un deuxième détecteur qui fonctionne selon l'effet photoacoustique.

3. Dispositif de mesure (20 ; 30) selon l'une des revendications précédentes,
**caractérisé en ce que**
le gaz combustible comporte au moins un troisième gaz et un quatrième gaz différent du troisième gaz, le troisième gaz et le quatrième gaz pouvant absorber la lumière infrarouge dans au moins une deuxième gamme de longueurs d'onde commune dans le spectre électromagnétique et le dispositif de mesure (20 ; 30) comportant une deuxième source de rayonnement infrarouge intermittent (22), une deuxième chambre d'échantillon (23) destinée à recevoir le gaz combustible, un troisième détecteur (24) qui comporte au moins le troisième gaz et qui fonctionne selon l'effet photoacoustique, et une deuxième chambre de filtrage (25) qui contient le quatrième gaz, et la deuxième chambre d'échantillon (23), le troisième détecteur (24) et la deuxième chambre de filtrage (25) étant disposés l'un par rapport à l'autre de manière à ce que la lumière infrarouge émise par la deuxième source de rayonnement infrarouge (22) traverse la deuxième chambre d'échantillon (23) et la deuxième chambre de filtrage (25) et est incidente au troisième détecteur (24) et le gaz combustible provenant de la sortie de fluide de la première chambre d'échantillon (3) pouvant être dirigé vers une entrée de fluide de la deuxième chambre d'échantillon (23).

4. Dispositif de mesure (30) selon la revendication 3,
**caractérisé en ce que**
le dispositif de mesure (30) comporte un quatrième détecteur (28) qui est disposé entre la deuxième source de rayonnement infrarouge (22) et le troisième détecteur (24) et qui comporte un cinquième gaz.

5. Dispositif de mesure (1 ; 10 ; 20 ; 30) selon l'une des revendications précédentes, **caractérisé en ce que**
le premier gaz est du propane et le deuxième gaz est du méthane.

6. Dispositif de mesure (1 ; 10 ; 20 ; 30) selon l'une des revendications précédentes,
**caractérisé en ce que**
le troisième gaz est l'éthane et le quatrième gaz est le propane.

7. Dispositif de mesure (1 ; 10 ; 20 ; 30) selon l'une des revendications précédentes,
**caractérisé en ce que**
le cinquième gaz est le dioxyde de carbone.

8. Procédé d'analyse d'un gaz combustible à l'aide du dispositif de mesure (1 ; 10 ; 20 ; 30) selon l'une des revendications 1 à 7, ledit procédé comprenant les étapes suivantes :
- introduire le gaz combustible dans la première chambre d'échantillon (3),
- générer un rayonnement infrarouge intermittent à l'aide la première source de rayonnement infrarouge (2),
- détecter une première intensité du rayonnement infrarouge à l'aide du premier détecteur (4),
- déterminer au moins une concentration du premier gaz en fonction de la première intensité du rayonnement infrarouge,
- transmettre des valeurs calculées à une unité de commande du brûleur (12) afin de réguler le brûleur (12).

9. Procédé selon la revendication 8 utilisant un dispositif de mesure (1 ; 10 ; 20 ; 30) selon l'une des revendications 1 à 7, ledit procédé comprenant les étapes supplémentaires suivantes :
- détecter une deuxième intensité du rayonnement infrarouge à l'aide du deuxième détecteur,
- déterminer au moins une deuxième concentration du deuxième gaz en fonction de la deuxième intensité du rayonnement infrarouge.

10. Procédé selon la revendication 8 ou 9 comprenant les étapes supplémentaires suivantes :
- introduire le gaz combustible de la première chambre d'échantillon (3) dans la deuxième chambre d'échantillon (23),
- générer un rayonnement infrarouge intermittent à l'aide de la deuxième source de rayonnement infrarouge (22),
- détecter une troisième intensité du rayonnement infrarouge à l'aide du troisième détecteur (24),
- déterminer au moins une troisième concentration du troisième gaz en fonction de la troisième intensité du rayonnement infrarouge,
- détecter une quatrième intensité du rayonnement infrarouge à l'aide de la deuxième chambre de filtrage (25), la deuxième chambre de filtrage (25) étant conçue comme un quatrième détecteur,
- déterminer au moins une quatrième concentration du quatrième gaz en fonction de la quatrième intensité du rayonnement infrarouge.

11. Procédé selon l'une des revendications 8, 9 et 10, une puissance calorifique ou un pouvoir calorifique du gaz combustible étant déterminé en fonction des première, deuxième, troisième et/ou quatrième concentrations.

12. Procédé selon l'une des revendications 8 à 11, un indice de Wobbe inférieur ou supérieur du gaz combustible étant déterminé en fonction des première, deuxième, troisième et/ou quatrième concentrations.
